(19) Europäisches Patentamt
European Patent Office
Office européen des brevets

(11) Veröffentlichungsnummer: **0 099 975**
**B1**

(12) # EUROPÄISCHE PATENTSCHRIFT

(45) Veröffentlichungstag der Patentschrift: **10.12.86**

(51) Int. Cl.⁴: **B 01 J 23/66,** C 07 D 301/10, C 07 C 87/06

(21) Anmeldenummer: **83105369.9**

(22) Anmeldetag: **31.05.83**

(54) **Silberkatalysatoren, Verfahren zu ihrer Herstellung und ihre Verwendung zur Herstellung von Ethylenoxid.**

(30) Priorität: **24.07.82 DE 3227753**
**24.03.83 DE 3310685**

(43) Veröffentlichungstag der Anmeldung: **08.02.84 Patentblatt 84/6**

(45) Bekanntmachung des Hinweises auf die Patenterteilung: **10.12.86 Patentblatt 86/50**

(84) Benannte Vertragsstaaten: **BE DE FR GB IT NL**

(56) Entgegenhaltungen:
**EP - A - 0 025 963**
**DE - A - 2 362 066**

(73) Patentinhaber: **HOECHST AKTIENGESELLSCHAFT,**
***Postfach 80 03 20, D-6230 Frankfurt am Main 80 (DE)***

(72) Erfinder: **Rebsdat, Siegfried, Dr., Trenbeckstrasse 24, D-8261 Burg Neuötting (DE)**
Erfinder: **Mayer, Sigmund, Hochfellnstrasse 20, D-8261 Burgkirchen/Alz (DE)**
Erfinder: **Alfranseder, Josef, Hauptstrasse 31, D-8261 Hofschallern Post Marktl (DE)**

**EP 0 099 975 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft Silberkatalysatoren, die aus Silber und Promotor auf einem hitzebeständigen, porösen Trägermaterial bestehen. Sie betrifft ferner ein Verfahren zur Herstellung dieser Katalysatoren und ihre Verwendung zur Herstellung von Ethylenoxid durch Oxidation von Ethylen mit Sauerstoff.

Ethylenoxid wird grosstechnisch durch Direktoxidation von Ethylen mit Sauerstoff über einem Silberkatalysator hergestellt. Eine allgemeine Beschreibung des Verfahrens findet sich in Kirk-Othmer: Encyclopedia of Chemical Technology, Band 9, Seiten 432 bis 471, John Wiley, London-NY, 1980.

Ein ethylen- und sauerstoffhaltiges Gas tritt oben in den Reaktor ein. Dieser besteht aus einem Bündel von mehreren tausend Rohren von 6 bis über 10 m Länge. Das Gas strömt über den in den Rohren befindlichen Katalysator, in der Regel bei einer Temperatur von 150 bis 400 °C und 0,15 bis 3 MPa Überdruck, wobei sich 5 bis 20% des eingesetzten Ethylens umsetzen.

Neben der Bildung von Ethylenoxid wird ein erheblicher Anteil des Ethylens, nämlich etwa 25%, zu Kohlendioxid und Wasser oxidiert (Totaloxidation), was die nachstehenden Reaktionsgleichungen veranschaulichen sollen:

Ethylenoxidbildung $2C_2H_4 + O_2 \rightarrow 2C_2H_4O$
Totaloxidation $C_2H_4 + 3O_2 \rightarrow 2CO_2 + 2H_2O$.

Zur Temperaturkontrolle und Abfuhr der Wärme befindet sich um die Rohre ein Wärmeträgermedium, das die freiwerdende Wärme aus dem Reaktor heraustransportiert. Das ethylenoxid- und kohlendioxidhaltige Reaktionsgas verlässt den Reaktor, um in den Aufarbeitungsteil zu gelangen, wo Ethylenoxid und Kohlendioxid abgetrennt werden. Das von Ethylenoxid und Kohlendioxid befreite Gas wird erneut mit Ethylen und Sauerstoff angereichert und wieder zum Reaktor gefördert. Es handelt sich um einen kontinuierlichen Kreisprozess mit heterogener Katalyse.

Als Katalysator dient ein Silberträgerkatalysator. Die Qualität eines derartigen Katalysators wird wesentlich durch seine Selektivität, seine Aktivität und seine Lebensdauer gekennzeichnet.

Die Selektivität ist der molare Prozentsatz an umgesetztem Ethylen, der zu Ethylenoxid reagiert.

Die Aktivität wird durch die Ethylenoxid-Konzentration am Reaktorausgang bei sonst konstanten Bedingungen (z.B. Temperatur, Druck, Gasmenge, Katalysatormenge) charakterisiert. Je höher die Ethylenoxid-Konzentration desto höher die Aktivität. Oder anders ausgedrückt: Je niedriger die zum Erreichen einer bestimmten Ethylenoxid-Konzentration erforderliche Temperatur desto höher die Aktivität.

Die Herstellung von Silberträgerkatalysatoren an sich ist schon seit langem bekannt. Sie werden bevorzugt nach dem folgenden Verfahren hergestellt:

Das Trägermaterial wird mit einer Lösung eines Silbersalzes getränkt.

Das getränkte Trägermaterial wird getrocknet, wobei sich das Silbersalz auf dem Trägermaterial niederschlägt. Das so mit Silbersalz imprägnierte Trägermaterial wird Bedingungen unterworfen, unter denen eine Zersetzung des Silbersalzes erfolgt, wobei sich elementares Silber bildet, das auf dem Träger in fein verteilter Form vorliegt. Dieser Schritt wird beispielsweise rein thermisch durch Erhitzen auf etwa 170 bis 400 °C oder mit Hilfe von Reduktionsmitteln wie Formaldehyd durchgeführt. Die thermische Reduktion kann unter Überleiten von inerten Gasen wie Luft, Stickstoff, Kohlendioxid oder Mischungen davon erfolgen.

In der Regel werden neben dem Silber auch sogenannte Promotoren wie Kalium-Rubidium und/oder Cäsium, auf das Trägermaterial aufgebracht, weil sie zu einer beträchtlichen Verbesserung der Wirksamkeit des fertigen Katalysators beitragen.

In neuerer Zeit ist festgestellt worden, dass Silberkatalysatoren mit hoher Wirksamkeit dann erreicht werden, wenn das Trägermaterial zum Aufbringen von Silber mit bestimmten Silbersalz-Amin-Komplexverbindung imprägniert worden ist. Diesbezüglich sind in der Zwischenzeit zahlreiche Vorschläge gemacht worden.

Nach der deutschen Auslegeschrift 2 159 346, die der US-Patentschrift 3 702 259 entspricht, wird das Trägermaterial mit einer wässrigen Lösung imprägniert, die mindestens ein Silbercarboxylat und zur Bildung eines Silbercarboxylat-Amin-Komplexes mindestens ein organisches Amin enthält, wobei das solubilisierende/reduzierende organische Amin vorzugsweise Ethylendiamin, ein Gemisch aus Ethylendiamin und Ethanolamin, aus Ammoniak und Ethylendiamin oder ein Gemisch aus Ammoniak und Ethanolamin ist.

Bei der Herstellung des aus der deutschen Offenlegungsschrift 2 300 512 bekannten Silberkatalysators, bestehend aus Silber in einer Menge von 3 bis 20 Gew.% und Kalium, Rubidium und/oder Cäsium als Promotor in einer Menge von 0,003 bis 0,05 Gew.% auf einem hitzebeständigen porösen Trägermaterial, Gewichtsprozente jeweils bezogen auf das Gewicht des Katalysators, wird zum Aufbringen des Silbers auf das Trägermaterial ebenfalls ein Silbercarboxylat-Ethylendiamin- und/oder -Ethanolamin-Komplex eingesetzt.

In der deutschen Offenlegungsschrift 2 622 228 und in der deutschen Auslegeschrift 2 640 540 werden als komplexbildende Amine für das Silbersalz, das organisch oder anorganisch sein kann, primäre Amine mit Alkylresten mit 1 bis 8 Kohlenstoffatomen, Polyamine der Alkane bis etwa zum Hexan, alicyclische Amine, wie Cyclohexylamin, heterocyclische Amine, wie Pyrrolidon, Piperidin und Morpholin, und insbesondere Amine der allgemeinen Formel (R, R')-CH-$NH_2$, wobei R und R' aliphatische Reste sind, genannt.

Aus den beiden deutschen Offenlegungsschriften 2 655 738 und 2 734 912 ist es bekannt, zur Abscheidung von Silber und dem Promotor Cäsium auf ein Trägermaterial eine Imprägnierlösung zu verwenden, die mindestens ein Silbercarboxylat, mindestens ein Amin und mindestens eine im Silbercarboxylat-Amin-Komplex lösliche Cäsiumverbindung enthält. Als Amine werden bestimmte aliphatische Diamine, aliphatische Polyamine und bestimmte aliphatische Aminoether eingesetzt. Die Imprägnierlösung kann auch Wasser enthalten.

In der US-Patentschrift 4 235 757 werden schliesslich Amine der Formel $NH_2$-R-NH-R′-OH, worin R und R′ eine Alkylgruppe mit 2 bis 4 Kohlenstoffatomen ist, als besonders vorteilhafte Komplexbilder zum Aufbringen des Silbers auf das Trägermaterial genannt.

Bezüglich des Aufbringens des Silbers und des Promotors auf das Trägermaterial werden in den genannten Druckschriften verschiedene Vorgangsweisen beschrieben. So werden nach den Verfahren der deutschen Offenlegungsschriften 2 300 512 und 2 734 912 das Silber (der Silbersalz-Aminkomplex) und der Promotor (die Promotorverbindung) gleichzeitig und mit einer Imprägnierung auf das Trägermaterial aufgebracht. Bei der Herstellung des Silberkatalysators nach der deutschen Auslegeschrift 2 640 540 ist die Art und Weise des Aufbringens des Silbers und des Promotors auf den Träger nicht kritisch. Silber und Promotor können gleichzeitig und in einem Imprägnierschritt aufgebracht werden oder auch nacheinander, wobei der Promotor in einem ersten und das Silber in einem zweiten Imprägnierschritt oder das Silber in einem ersten und der Promotor in einem zweiten Imprägnierschritt niedergeschlagen werden.

Der Stand der Technik über die Herstellung von Silberkatalysatoren mit verbesserten Eigenschaften unter Verwendung von Silbersalz-Amin-Komplexen zeigt also, dass bereits eine grosse Zahl vom komplexbildenden Aminen empfohlen worden ist. Es besteht aber dennoch ein Bedarf nach weiteren derartigen Vorschlägen, zumal die bekannten Amin-Komplexbildner einerseits häufig relativ schwer zugänglich sind und andererseits die damit erzielte Verbesserung der Aktivität, Selektivität und der Lebensdauer noch zu wünschen übrig lässt.

Es wurde nun überraschenderweise gefunden, dass als komplexbildendes (solubilisierend/reduzierend wirkendes) Amin ein Gemisch aus mindestens einem tert.-Alkylamin (tert. = tertiärem) und mindestens einem sek.-Alkylamin (sek. = sekundärem) besonders vorteilhaft ist. Damit werden nämlich Silberkatalysatoren erhalten, die nicht nur eine unerwartet hohe Aktivität, Selektivität und Lebensdauer besitzen, sondern überraschenderweise auch eine derart geringe Abhängigkeit der Selektivität vom Ethylenumsatz zeigen, wie sie mit den aus dem Stand der Technik bekannten Aminen nicht erreicht werden kann.

Die Wirkung des vorgeschlagenen Amins (Amingemischs) ist unabhängig von der Art und Weise des Aufbringens von Silber und Promotor auf den Träger.

Der erfindungsgemässe Silberkatalysator, bestehend aus Silber in einer Menge von 3 bis 20 Gew.% und Kalium, Rubidium und/oder Cäsium als Promotor in einer Menge von 0,003 bis 0,05 Gew.% auf einem hitzebeständigen, porösen Trägermaterial, Gewichtsprozente jeweils bezogen auf das Gewicht des Katalysators (Gewicht des fertigen Katalysators oder Gesamtgewicht des Katalysators), wobei das Silber und der Promotor durch Imprägnieren des Trägermaterials mit Silbersalz-Amin-Komplexen und mit Promotorverbindungen, und durch Trocknen und Erhitzen des imprägnierten Trägermaterials auf 170 bis 400°C zur Zersetzung des Silbersalz-Amin-Komplexes in metallisches Silber aufgebracht worden sind, ist dadurch gekennzeichnet, dass als Amin zur Bildung des Silbersalz-Amin-Komplexes ein Gemisch aus a) 10 bis 40 Gew.% von mindestens einem tert.-Alkylamin der allgemeinen Formel I

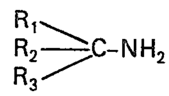

und b) 60 bis 90 Gew.% von mindestens einem sek.-Alkylamin der allgemeinen Formel II

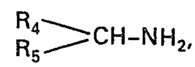

worin $R_1$, $R_2$, $R_3$, $R_4$ und $R_5$ eine Alkylgruppe mit 1 bis 4 Kohlenstoffatomen bedeuten, eingesetzt worden ist.

Das erfindungsgemässe Verfahren, bei dem auf einem hitzebeständigen, porösen Trägermaterial Silber in einer Menge von 3 bis 20 Gew.% und Kalium, Rubidium und/oder Cäsium als Promotor in einer Menge von 0,003 bis 0,05 Gew.% aufgebracht werden, Gewichtsprozente jeweils bezogen auf das Gewicht des Katalysators (Gewicht des fertigen Katalysators oder Gesamtgewicht des Katalysators), wobei zum Aufbringen des Silbers und des Promotors das Trägermaterial mit Silbersalz-Amin-Komplexen und mit Promotorverbindungen imprägniert wird und das imprägnierte Trägermaterial zur Zersetzung des Silbersalz-Amin-Komplexes in metallisches Silber auf 170 bis 400°C erhitzt wird, ist dadurch gekennzeichnet, dass als Amin zur Bildung des Silbersalz-Amin-Komplexes ein Gemisch aus a) 10 bis 40 Gew.% von mindestens einem tert.-Alkylamin der allgemeinen Formel I

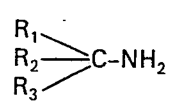

und b) 60 bis 90 Gew.% von mindestens einem sek.-Alkylamin der allgemeinen Formel II

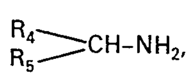

worin $R_1$, $R_2$, $R_3$, $R_4$ und $R_5$ eine Alkylgruppe mit 1 bis 4 Kohlenstoffatomen bedeuten, eingesetzt wird.

Das erfindungsgemässe solubilisierende/reduzierende Amingemisch besteht vorzugsweise aus 20 bis 30 Gew.% von mindestens einem tert.-Alkylamin der Formel I und 70 bis 80 Gew.% von mindestens einem sek.- Alkylamin der Formel II.

Von den erfindungsgemäss einzusetzenden tert.-Alkylaminen und sek.-Alkylaminen sind jene bevorzugt, die Methyl ($-CH_3$) oder Ethyl ($-C_2H_5$) als Alkylgruppe $R_1$ bis $R_3$ beziehungsweise $R_4$ und $R_5$ haben. Als tert.-Alkylamin ist das tert.-Butylamin, nämlich

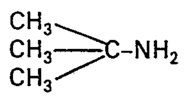

und als sek.-Alkylamin das sek.-Butylamin, nämlich

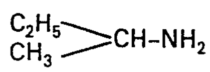

besonders bevorzugt.

Das erfindungsgemässe komplexbildende Amin wird mindestens in einer solchen Menge eingesetzt, die zur Bildung des Silbersalz-Amin-Komplexes erforderlich ist, durch die also das verwendete Silbersalz vollständig gelöst wird. Da ein Silberkation gemäss Reaktionsgleichung $Ag(amin)_2X$ (X Anion des eingesetzten Silbersalzes) stöchiometrisch zwei Aminogruppen bindet, ist der Einsatz von mindestens zwei Aminogruppen pro mol Silberkation erforderlich. Um vollständige Löslichkeit zu gewährleisten, wird deswegen in der Regel ein geringer molarer Überschuss an solubilisierendem Amin genommen.

Die erfindungsgemässe Amin-Menge liegt im allgemeinen bei 2,05 bis 5 mol, vorzugsweise 2,1 bis 3 mol Amin pro mol Silber in der eingesetzten Silberverbindung.

Beim neuen Silberkatalysator beträgt die Silbermenge vorzugsweise 7 bis 14 Gew.% und die Promotormenge (Kalium Rubidium oder Cäsium oder eine Mischung davon) vorzugsweise 0,008 bis 0,035 Gew.%, Gewichtsprozente jeweils bezogen auf das Gewicht des Katalysators. Der Promotor ist vorzugsweise Cäsium.

Die Art des Silbersalzes und der Promotorverbindung für die Imprägnierung des Trägers ist an sich nicht kritisch.

Als Silbersalze können anorganische und/oder organische Salze eingesetzt werden. Zweckmässige organische Salze sind die Silbercarboxylate wie Silberformiat, -acetat, -malonat, -oxalat, -lactat und/oder Silbercitrat.

Zweckmässige anorganische Salze sind Silbercarbonat, -nitrat und/oder Silbernitrit. Bevorzugt unter den genannten Silbersalzen sind das Silberacetat, -lactat, -oxalat, -carbonat und/oder Silbernitrat; sie sind leicht zugänglich und relativ billig.

Als Promotorverbindungen können anorganische und/oder organische Verbindungen eingesetzt werden, wobei Salze und Hydroxide bevorzugt sind. Das Anion der Promotorverbindung ist nicht kritisch, da es für die katalytische Wirksamkeit ohne Bedeutung ist; der Promotor wirkt in Form des Kations in der aufgebrachten Promotorverbindung. Zweckmässige Promotorsalze sind die Carboxylate, wie das Formiat, Acetat, Malonat, Oxalat, Lactat, Tartrat und/oder Citrat. Zweckmässige anorganische Salze sind das Carbonat, Bicarbonat, Phosphat, Nitrat und/oder Nitrit. Bevorzugt unter den genannten Promotorverbindungen sind das leicht zugängliche und relativ billige Acetat und/oder Nitrat.

Als Trägermaterial der erfindungsgemässen Silberkatalysatoren kommen die üblichen, im Handel erhältlichen hitzebeständigen und porösen Materialien in Betracht. Diese sind auch in Gegenwart der bei der Oxidation von Ethylen herrschenden Reaktionsbedingungen und anwesenden chemischen Verbindungen inert. Das Trägermaterial zum Herstellen der erfindungsgemässen Silberkatalysatoren ist nicht kritisch, geeignete Träger sind beispielsweise Kohle, Korund, Siliciumcarbid, Siliciumdioxid, Aluminiumoxid und Gemische aus Aluminiumoxid und Siliciumdioxid. Bevorzugt ist α-Aluminiumoxid, da es einen weitgehend gleichmässigen Porendurchmesser aufweist. Es besitzt eine spezifische Oberfläche von 0,1 bis 1 $m^2/g$, vorzugsweise 0,2 bis 0,6 $m^2/g$ (gemessen nach der bekannten B.E.T.-Methode), ein spezifisches Porenvolumen von 0,1 bis 1 $cm^3/g$, vorzugsweise 0,2 bis 0,6 $cm^3/g$ (gemessen nach der bekannten Quecksilber- oder Wasseradsorptionsmethode), eine scheinbare Porosität von 20 bis 70 Vol.% vorzugsweise 40 bis 60 Vol.% (gemessen nach der bekannten Quecksilber- oder Wasseradsorptionsmethode), einen mittleren Porendurchmesser von 0,3 bis 15 µm, vorzugsweise 1 bis 10 µm, und einen prozentuellen Anteil an Poren mit einem Durchmesser von 0,03 bis 10 µm von mindestens 50 Gew.% (Porendurchmesser und Porendurchmesserverteilung werden bekanntlich aus der spezifischen Oberfläche und der scheinbaren Porosität ermittelt). Das Trägermaterial wird vorteilhafterweise in Form von Granulaten, Kugeln, Ringen, Pellets und dergleichen eingesetzt. Beispiele von bevorzugten α-Aluminiumoxid- oder α-Aluminiumoxid enthaltenden Trägermaterialien sind die von der Firma Norton Company, USA, angebotenen Typen mit der Bezeichnung SA 5551 und SA 5552 oder die SAHM-Typen der Firme United Catalyst, USA.

Das Aufbringen von Silber und Promotor auf das Trägermaterial erfolgt mit Hilfe des bekannten Imprägnierverfahrens. Dabei wird das Trägermaterial mit der Imprägnierlösung in Kontakt gebracht, vorzugsweise getränkt (durch Eintauchen oder Übergiessen), wobei die Lösung durch Absorption und/oder durch Kapillarwirkung in die Poren des Trägermaterials eindringt. Die überschüssige Imprägnierlösung wird dann abgetrennt (zum Beispiel durch Abgiessen, Abtropfen, Abfiltrieren oder Abzentrifugieren), worauf das vollgesogene Trägermaterial getrocknet wird und wobei sich Silberverbindung und/oder Promotorverbindung abscheiden.

Die Menge an Imprägnierlösung wird im allgemeinen so gewählt, dass ein volumenmässiger Überschuss, bezogen auf das zu imprägnierende Trägermaterialvolumen, vorliegt. Im allgemeinen wird das 0,5- bis 3fache, vorzugsweise das 1- bis 2fache Volumen an Imprägnierflüssigkeit, bezogen auf das Volumen an Trägermaterial, genommen. Die Imprägnierzeit, das ist die Zeit, in der das Trägermaterial mit der Imprägnierflüssigkeit in Kontakt bleibt, ist klarerweise so zu wählen, dass die erforderliche Menge an abzuscheidender Silberverbindung und Promotorverbindung auf den Träger aufgebracht wird. Sie beträgt im allgemeinen 3 bis 60 Minuten und hängt insbesondere von der Konzentration der Imprägnierlösung an Silber- und Promotorverbindung, von dem verwendeten Trägermaterial und von dessen jeweiligem Saugvermögen ab. Die beim Imprägnieren angewandte Temperatur kann innerhalb weiter Grenzen variieren. Im allgemeinen wird bei Raumtemperatur imprägniert. Es können auch höhere Temperaturen angewandt werden. Die Imprägniertemperatur beträgt demnach in der Regel 15 bis 80°C, vorzugsweise 20 bis 50°C. Die Imprägnierung wird in der Regel bei Atmosphärendruck durchgeführt. Um den Imprägniervorgang zu beschleunigen, kann die Imprägnierung auch im Vakuum vorgenommen werden.

Geeignete Lösungsmittel zur Bereitung der Imprägnierlösungen sind Wasser, organische Flüssigkeiten und Gemische aus Wasser und organischen Flüssigkeiten. Bevorzugte Lösungsmittel sind Wasser, Methanol, Ethanol, Propanol, Isopropanol und Aceton sowie Gemische davon.

Die Trocknung des von der überschüssigen Imprägnierflüssigkeit abgetrennten imprägnierten Trägermaterials erfolgt im allgemeinen bei einer Temperatur von 20 bis 150°C, vorzugsweise 50 bis 120°C. Die Abdampfung des Lösungsmittels kann beispielsweise mit Hilfe von Hordentrocknern, Drehrohröfen oder durch Überleiten von heissen Inertgasen wie Stickstoff, Luft und/oder Kohlendioxid, durchgeführt werden. Die Temperatur richtet sich im allgemeinen nach dem Siedepunkt des Lösungsmittels der Imprägnierflüssigkeit. Nach einer bevorzugten Arbeitsweise wird die Trocknung in Gegenwart von Inertgas und bei einer Temperatur von 50 bis 120°C durchgeführt.

Das getrocknete, imprägnierte Trägermaterial wird zur Umwandlung (Reduzierung, thermischen Zersetzung) des abgeschiedenen Silbersalz-Amin-Komplexes in metallisches Silber im allgemeinen auf eine Temperatur von 170 bis 400°C, vorzugsweise auf eine Temperatur von 200 bis 350°C, erhitzt. Das Erhitzen auf diese Temperaturen kann beispielsweise in einem Hordentrockner, Drehrohrofen, elektrisch beheizten Rohr oder durch Überleiten eines entsprechend erhitzten Inertgases wie Luft, Stickstoff, Kohlendioxid oder einer Mischung davon, durchgeführt werden. Die Überführung des Silber-Amin-Komplexes in metallisches Silber kann auch mit Hilfe von überhitztem Wasserdampf vorgenommen werden. Nach einer bevorzugten Arbeitsweise wird die thermische Zersetzung der aufgebrachten Silberverbindung durch Überleiten von Luft, Stickstoff und/oder Kohlendioxid über das auf 200 bis 350°C erhitzte, fertig imprägnierte Trägermaterial (Vorkatalysator) durchgeführt. Die bei den genannten Temperaturen erforderliche Erhitzungszeit beträgt im allgemeinen 0,05 bis 5 Stunden, vorzugsweise 0,1 bis 1 Stunde. Durch die Zersetzung der Silberverbindung bildet sich ein festhaftender Niederschlag von metallischen Silberteilchen auf dem Trägermaterial (die Promotorverbindungen werden nicht zum entsprechenden Metall reduziert, die Alkalimetalle Kalium, Rubidium und Cäsium liegen also im wesentlichen in Form ihrer Kationen und nicht als freies Alkalimetall vor). Das Silber (die Silberteilchen) liegt in der Regel in Form von festhaftenden, im wesentlichen gleichmässig verteilten, nicht zusammenhängenden, diskreten Teilchen vor.

Die Art und Weise des Aufbringens der Silbermenge (in Form des eingesetzten Silbersalz-Amin-Komplexes) und der Promotormenge (in Form der eingesetzten Promotorverbindungen) im Rahmen des Imprägnierverfahrens ist nicht kritisch. So können Silber und Promotor gleichzeitig oder zeitlich verschieden, das heisst nacheinander in beliebiger Reihenfolge, auf den Träger aufgebracht werden. Bei der gleichzeitigen Abscheidung von Silber und Promotor kann die gesamte Silber- und Promotormenge mit einer Imprägnierung aufgebracht werden. Es können mehrere Imprägnierungen, vorzugsweise zwei, angewandt werden, wobei pro Imprägnierung nur ein Teil der gesamten Silber- und Promotormenge aufgebracht wird. In diesem Fall hat es sich als vorteilhaft erwiesen, in einem ersten Imprägnierschritt 55 bis 85 Gew.% von der gesamten Silbermenge und 15 bis 45 Gew.% von der gesamten Promotormenge gleichzeitig auf den Träger aufzubringen und nach Trocknung in einem zweiten Imprägnierschritt die jeweiligen Restmengen. Bei der Nacheinanderabscheidung von Silber und Promotor kann zuerst das Silber und dann der Promotor oder zuerst der Promotor und dann das Silber aufgebracht werden, und zwar mit jeweils einer oder mit mehreren Imprägnierungen. Wird zuerst die Silberverbindung und dann die Promotorverbindung aufgebracht, so kann vor dem Aufbringen der Promotorverbindung zu metallischem Silber reduziert werden, es kann aber auch nur eine Zwischentrocknung vorgenommen werden. Nach einer weiteren Methode kann in einem Imprägnierschritt die gesamte Silbermenge und nur ein Teil der gesamten Promotormenge und in einem zweiten Imprägnierschritt die restliche Promotormenge aufgebracht werden. In diesem Fall hat es sich als vorteilhaft erwiesen, im ersten Imprägnierschritt die gesamte Silbermenge und 15 bis 45 Gew.% von der gesamten Promotormenge abzuscheiden und nach Reduktion zu metallischem Silber in einem zweiten Imprägnierschritt den Rest auf die gesamte Promotormenge abzuscheiden. Analog dazu

kann in einem ersten Imprägnierschritt die gesamte Promotormenge und nur ein Teil der gesamten Silbermenge und in einem zweiten Imprägnierschritt die restliche Silbermenge aufgebracht werden. Es kann ferner beispielsweise auch so vorgegangen werden, dass Silber und Promotor nacheinander in abwechselnder Reihenfolge in mehreren Imprägnierungen auf dem Träger aufgebracht werden. So kann zum Beispiel zunächst ein Teil der gesamten Silbermenge, anschliessend in einer oder mehreren Imprägnierungen die Promotormenge und darauf die restliche Silbermenge niedergeschlagen werden. Analog dazu kann mit dem Promotor begonnen werden.

Die Imprägnierlösungen für die gewählte Imprägniermethode können hinsichtlich Konzentration an Silber- und Promotorverbindung innerhalb weiter Grenzen variieren. Diese Konzentrationen sind bekanntlich insbesondere durch den gewünschten Gehalt an Silber und Promotor auf dem Träger bestimmt. Die Konzentration an Silber und Promotor in den Imprägnierlösungen ist lediglich so zu wählen, dass durch die Imprägnierung 3 bis 20 Gew.%, vorzugsweise 7 bis 14 Gew.%, Silber und 0,003 bis 0,05 Gew.%, vorzugsweise 0,008 bis 0,035 Gew.%, Promotor abgeschieden werden kann. Durch Vorversuche und analytische Bestimmung der auf dem Träger niedergeschlagenen Menge an Silber- und Promotorverbindung kann die zweckmässige Konzentration leicht und schnell ermittelt werden. So besteht beispielsweise eine zweckmässige Imprägnierlösung für das gleichzeitige Aufbringen von Silber und Promotor auf dem Träger mit einer oder mehreren Imprägnierungen im wesentlichen aus (a) Wasser, (b) 30 bis 40 Gew.% Silberverbindung, (c) 2,1 bis 3 mol Amin vom neuen Amingemisch pro mol Silber in der eingesetzten Silberverbindung und (d) 0,03 bis 0,1 Gew.% Promotorverbindung, Gewichtsprozente jeweils bezogen auf das Gewicht der Lösung. Für den Silberauftrag und für den Promotorauftrag mit jeweils einer oder mehreren Imprägnierungen sind beispielsweise die folgenden Lösungen geeignet: (a) Wasser, (b) 30 bis 40 Gew.% Silberverbindung und (c) 2,1 bis 3 mol Amin vom neuen Amingemisch pro mol Silber in der eingesetzten Silberverbindung beziehungsweise (a) Wasser oder ein Gemisch aus Wasser und einem organischen Lösungsmittel, vorzugsweise Methanol, Ethanol, Propanol, Isopropanol und Aceton, und (b) 0,03 bis 0,1 Gew.% Promotorverbindung, Gewichtsprozente jeweils bezogen auf das Gewicht der Lösung (in diesen Imprägnierlösungen ist also das Lösungsmittel in einer solchen Menge vorhanden, dass es die angegebene Menge der anderen Komponenten auf 100 Gew.% ergänzt).

Der erfindungsgemässe Silberkatalysator besitzt eine hohe Aktivität und Selektivität und eine lange Lebensdauer. Was ihn besonders auszeichnet, ist die überraschend geringe Abhängigkeit der Selektivität vom Ethylenumsatz.

Die Selektivität der Ethylenoxidbildung eines Silberkatalysators hängt bekanntlich von der im Reaktor umgesetzten Ethylenmenge ab, und zwar in der Weise, dass eine Erhöhung des Ethylenumsatzes zu einer Erniedrigung der Selektivität führt. Diese je nach Silberkatalysator mehr oder weniger grosse Abhängigkeit stellt einen erheblichen Nachteil dar. In dem wirtschaftlichen Bestreben, eine Ethylenoxid-Anlage mit immer höherer Auslastung, das heisst mit einem immer höheren Ethylenumsatz zu betreiben, muss nämlich zwangsläufig eine sinkende Selektivität und damit ein zunehmender Ethylenverlust in Kauf genommen werden. Ein Silberkatalysator ist demnach umso besser je geringer der Selektivitätsabfall bei steigendem Ethylenumsatz ist.

Der erfindungsgemässe Silberkatalysator weist im Vergleich zu den Silberkatalysatoren des Standes der Technik eine beträchtlich geringere Abhängigkeit der Selektivität vom Ethylenumsatz auf. Damit verbunden ist der weitere grosse Vorteil, dass die nötige Katalysatormenge im Vergleich zu den bekannten Silberkatalysatoren relativ klein gehalten werden kann. Dies ist bei dem hohen Preis der Silberkatalysatoren klarerweise ein erheblicher wirtschaftlicher Vorteil. Mit dem erfindungsgemässen Silberkatalysator kann ferner mit einer relativ kleinen Gasmenge gearbeitet werden, woraus eine Energieeinsparung im Zusammenhang mit der Verdichtung des Gases resultiert.

Beim Einsatz des erfindungsgemässen Silberkatalysators wird also mit einer relativ kleinen Gasmenge und einem relativ hohen Ethylenumsatz noch eine gute Selektivität erzielt, wobei all die weiteren oben angeführten Vorteile dazukommen.

Das erfindungsgemässe Verfahren zur Herstellung der neuen Silberkatalysatoren ist einfach und leicht durchzuführen. Es enthält keine komplizierten oder aufwendigen Verfahrensschritte, und es sind auch keine speziellen Imprägnierlösungen erforderlich. Die erfindungsgemäss einzusetzenden Amine sind leicht zugänglich, leicht zu handhaben und billig.

Die Verwendung des neuen Silberkatalysators erfolgt unter an sich bekannten Bedingungen, wie Temperatur, Druck, Verweilzeit, Verdünnungsmittel, Bremssubstanzen zur Kontrolle der katalytischen Oxidation von Ethylen mit Sauerstoff, Recycling, verfahrenstechnische Massnahmen zur Erhöhung der Ethylenoxidausbeute und dergleichen. Die Reaktionstemperatur liegt im allgemeinen bei 150 bis 400°C, vorzugsweise 175 bis 250°C, der Reaktionsdruck bei 0,15 bis 3 MPa, vorzugsweise 1 bis 2 MPa. Die eingesetzte Beschickungsmischung enthält im allgemeinen 5 bis 30 Mol-% Ethylen, 3 bis 15 Mol-% Sauerstoff und als Rest inerte Gase, wie Stickstoff, Kohlendioxid, Wasserdampf, Methan, Ethan, Argon und dergleichen, sowie Vinylchlorid, 1,2-Dichlorethan und dergleichen als Bremssubstanzen. Aus dem Umsetzungsprodukt wird das Ethylenoxid in üblicher Weise isoliert und das Gasgemisch wie üblich eventuell gereinigt und wieder zurückgeführt. Nach einer bevorzugten Aufführungsform der Verwendung der erfindungsgemässen Silberkatalysatoren wird Ethylenoxid durch Oxidation von Ethylen mit einem etwa 8,5 Gew.% Sauerstoff enthaltenden Gasgemisch bei einer Temperatur von 175 bis 250°C in Gegenwart des neuen Silberkatalysators hergestellt.

Die Erfindung wird nun an Beispielen noch näher erläutert.

Beispiel 1

Zur Herstellung eines erfindungsgemässen Katalysators wurde eine Lösung aus

| | |
|---|---|
| 21,000 g (26,5655 Gew.%) | Wasser |
| 30,000 g (37,9502 Gew.%; 0,177 mol Silber) | Silbernitrat |
| 7,000 g (8,8555 Gew.%; 0,096 mol) | tert.-Butylamin |
| 21,000 g (26,5655 Gew.%; 0,287 mol) | sek.-Butylamin |
| 0,050 g (0,0633 Gew.%) | Cäsiumnitrat |

bereitet (es wurden also 0,383 mol erfindungsgemässes Amin mit 25 Gew.% tert.-Butylamin und 75 Gew.% sek.-Butylamin für 0,177 mol Silber, das sind 2,16 mol dieses Amins pro mol Silber, eingesetzt).

In diese Lösung wurde das Trägermaterial SAHM der Firma United Catalyst, nämlich α-Aluminiumoxid in Form von Zylindern mit einer spezifischen Oberfläche von 0,2 $m^2/g$ 15 Minuten lang bei 40°C vollständig eingetaucht. Nach dem Abtropfen der überschüssigen Imprägnierlösung wurde das feuchte Trägermaterial 30 Minuten lang bei 110°C in einer Luft-Stickstoff-Atmosphäre getrocknet.

25 g des so getränkten und getrockneten Trägers wurden anschliessend zur Reduktion der auf dem Träger befindlichen Silberverbindung in ein auf 200°C vorgeheiztes Glasrohr gefüllt. Das Rohr mit 30 mm lichter Weite und 300 mm Länge wurde von 30 l Luft pro Stunde von unten her durchströmt. Etwa 50 mm über dem zu reduzierenden Träger wurde zur Verhinderung einer möglichen Explosion 60 l Stickstoff pro Stunde eingeblasen. Nach halbstündiger Reduktion lag der fertige, einsatzbereite Silberkatalysator vor, der 8,7 Gew.% Silber und 0,014 Gew.% Cäsium enthielt.

20 ml dieses Katalysators wurden in einem Glasreaktor bei Atmosphärendruck mit einem Ethylen-Sauerstoffgemisch bei 185°C zur Reaktion gebracht. Das eingesetzte Gas enthielt 30 Vol.-% Ethylen, 8 Vol.-% Sauerstoff, 0,0003 Vol.-% Vinylchlorid, Rest Stickstoff. Die Belastung des Katalysators betrug 400 Liter Gas pro Liter Katalysator pro Stunde.

Das den Reaktor verlassende Reaktionsprodukt enthielt 1,8 Vol.-% Ethylenoxid. Aus der analytischen Ermittlung des gebildeten Ethylenoxids, Kohlendioxids und der Menge des umgesetzten Ethylens wurde die Selektivität (mol gebildetes Ethylenoxid pro mol umgesetztes Ethylen) sowie der Ethylenumsatz (Vol.-% umgesetztes Ethylen, bezogen auf eingesetztes Ethylen) errechnet. Es ergab sich eine Selektivität von 80,7% bei 7,7% Ethylenumsatz.

Nach 2 Monaten Versuchsdauer wurde noch immer kein Abfall der Selektivität festgestellt.

Um die Abhängigkeit der Selektivität dieses Katalysators vom Ethylenumsatz zu prüfen, wurde die Reaktionstemperatur von 185°C auf 192°C erhöht. Der Ethylenumsatz lag nun bei 12% (vorher 7,7%) und die Menge an gebildetem Ethylenoxid bei 2,9 Vol.-% (vorher 1,8 Vol.-%). Es ergab sich eine Selektivität von 79,0%. Trotz einer Steigerung des Ethylenumsatzes um 4,3 Einheiten sank also die Selektivität um nur 1,7 Einheiten.

Beispiel 2

Zur Herstellung eines erfindungsgemässen Katalysators wurde eine Lösung aus

| | |
|---|---|
| 21,000 g (26,9058 Gew.%) | Wasser |
| 30,000 g (38,4369 Gew.%; 0,179 mol Silber) | Silberacetat |
| 5,000 g (6,4061 Gew.%; 0,068 mol) | tert.-Butylamin |
| 22,000 g (28,1871 Gew.%; 0,301 mol) | sek.-Butylamin |
| 0,050 g (0,0641 Gew.%) | Cäsiumacetat |

bereitet (es wurden also 0,369 mol erfindungsgemässes Amin mit 18,5 Gew.% tert.-Butylamin und 81,5 Gew.% sek.-Butylamin für 0,179 mol Silber, das sind 2,06 mol dieses Amins pro mol Silber, eingesetzt).

Es wurde das Trägermaterial SA 5552 der Firma Norton eingesetzt, ein α-Aluminiumoxid in Form von Zylindern mit einer spezifischen Oberfläche von 0,3 $m^3/g$. Die Tränkung bei 50°C 15 Minuten lang, die Trocknung und die Reduzierung erfolgten wie im Beispiel 1. Erhalten wurde ein Silberkatalysator mit 8,8 Gew.% Silber und 0,015 Gew.% Cäsium.

Dieser Katalysator produzierte unter den Testbedingungen des Beispiels 1 bei 183°C 1,8 Vol.-% Ethylenoxid. Aus der analytisch ermittelten Menge an Ethylenoxid, Kohlendioxid und umgesetztem Ethylen wurde eine Selektivität von 80,5% bei 7,9% Ethylenumsatz errechnet.

Bei Anhebung der Temperatur auf 190°C ergaben sich 2,8 Vol.-% Ethylenoxid und eine Selektivität von 79% bei 11,7% Ethylenumsatz. Trotz einer Steigerung des Ethylenumsatzes um 3,8 Einheiten sank also die Selektivität um nur 1,5 Einheiten.

Nach 2 Monaten Versuchsdauer unter diesen Bedingungen wurde noch immer kein Selektivitätsabfall festgestellt.

Beispiel 3 (Vergleichsbeispiel)

In diesem Beispiel wurde, verglichen mit den erfindungsgemässen Beispielen, kein tert.-Butylamin eingesetzt.

Es wurde wie im Beispiel 2 vorgegangen. Die Imprägnierlösung bestand aus

| | |
|---|---|
| 21,000 g (26,5655 Gew.%) | Wasser |
| 30,000 g (37,9505 Gew.%; 0,177 mol Silber) | Silbernitrat |

| | |
|---|---|
| 28,000 g (35,4207 Gew.%; 0,383 mol) | sek.-Butylamin |
| 0,050 g (0,0633 Gew.%) | Cäsiumacetat |

Der erhaltene Silberkatalysator hatte 8,6 Gew.% Silber und 0,015 Gew.% Cäsium.

Dieser Katalysator produzierte unter den Testbedingungen des Beispiels 1 bei 193°C 1,8 Vol.-% Ethylenoxid. Es wurde eine Selektivität von 79,5% bei 8% Ethylenumsatz errechnet. Nach Erhöhung der Reaktionstemperatur auf 195°C wurden 2,7 Vol.-% Ethylenoxid produziert, und es wurde eine Selektivität von 77% bei 12% Ethylenumsatz festgestellt. Durch die Steigerung des Ethylenumsatzes um 4 Einheiten sank die Selektivität um 2,5 Einheiten. Nach 2 Monaten Versuchsdauer unter diesen Bedingungen sank die Selektivität weiter um 0,3 Einheiten.

Beispiel 4 (Vergleichsbeispiel)

Beispiel 2 wurde wiederholt mit dem Unterschied, dass anstelle des erfindungsgemässen Amingemisches ein Gemisch aus 5,0 g Ethanolamin und 12,0 g Ethylendiamin gemäss der deutschen Patentschrift 2 159 346 (die der US-Patentschrift 3 702 259 äquivalent ist) eingesetzt wurde.

Die Imprägnierlösung bestand aus

| | |
|---|---|
| 21,000 g (30,8597 Gew.%) | Wasser |
| 30,000 g (44,0852 Gew.%; 0,179 mol Silber) | Silberacetat |
| 5,000 g (7,3475 Gew.%; 0,082 mol) | Ethanolamin |
| 12,000 g (17,6341 Gew.%; 0,200 mol) | Ethylendiamin |
| 0,050 g (0,0735 Gew.%) | Cäsiumacetat |

(Die 0,082 mol Ethanolamin entsprechen 0,46 mol Ethanolamin pro mol Silber und die 0,200 mol Ethylendiamin entsprechen 1,12 mol Ethylendiamin pro mol Silber; das Ethylendiamin enthält zwei Aminogruppen.)

Der Test dieses Katalysators erfolgte wie in Beispiel 1. Nachstehend ist das Ergebnis des Testes zusammengefasst.

| Temperatur °C | Ethylenumsatz % | Selektivität % |
|---|---|---|
| 192 | 7 | 78,5 |
| 196 | 11 | 75,5 |

Beispiel 5 (Vergleichsbeispiel)

Beispiel 2 wurde wiederholt mit dem Unterschied, dass anstelle des erfindungsgemässen Amingemisches 30,0 g des Amins $H_2N-CH_2CH_2-NH-CH_2CH_2-OH$ gemäss der US-Patentschrift 4 235 757 eingesetzt wurden.

Die Imprägnierlösung bestand aus

| | |
|---|---|
| 21,000 g (25,9099 Gew.%) | Wasser |
| 30,000 g (37,0142 Gew.%; 0,179 mol Silber) | Silberacetat |
| 30,000 g (37,0142 Gew.%; 0,289 mol) $H_2N-CH_2CH_2-NH-CH_2CH_2-OH$ | |
| 0,050 g (0,0617 Gew.%) | Cäsiumacetat. |

(Die 0,289 mol des Amins entsprechen 1,61 mol Amin pro mol Silber; dieses Amin enthält zwei komplexbildende Aminogruppen.)

Der Test dieses Katalysators erfolgt wie in Beispiel 1. Nachstehend ist das Ergebnis des Testes zusammengefasst.

| Temperatur °C | Ethylenumsatz % | Selektivität % |
|---|---|---|
| 192 | 7 | 79 |
| 196 | 10 | 76,5 |

Beispiel 6

Zur Herstellung eines erfindungsgemässen Katalysators wurden wie in Beispiel 1 beschrieben, 91,5 g Aluminiumoxidringe in eine Lösung aus

| | |
|---|---|
| 10,500 g (26,5823 Gew.%) | Wasser |
| 15,000 g (37,9747 Gew.%; 0,088 mol Silber) | Silbernitrat |
| 3,800 g (9,6203 Gew.%; 0,052 mol) | tert.-Butylamin |
| 10,200 g (25,8227 Gew.%; 0,0139 mol) | sec.-Butylamin |

bei 40°C 5 Minuten lang getaucht (es wurden also 0,0191 mol erfindungsgemässes Amin aus 27 Gew.% tert.-Butylamin und 73 Gew.% sek.-Butylamin für 0,088 mol Silber, das sind 2,16 mol dieses Amins pro mol Silber, eingesetzt). Nach dem Abtropfen der überschüssigen Imprägnierlösung wurde 30 Minuten lang bei 110°C getrocknet. Der so getränkte und getrocknete Träger wurde anschliessend drei Minuten lang bei 40°C in eine Lösung aus

| | |
|---|---|
| 0,670 g (0,0670 Gew.%) | Cäsiumnitrat |
| 994,330 g (99,4330 Gew.%) | Methanol |
| 5,000 g (0,500 Gew.%) | Wasser |

getaucht und nach dem Abtropfen der überschüssigen Lösung 15 Minuten lang bei 110°C getrocknet. Die Reduktion erfolgte wie in Beispiel 1 beschrieben.

Der fertige Katalysator, bei dessen Herstellung zuerst das gesamte Silber (erste Imprägnierung) und anschliessend (zweite Imprägnierung) das gesamte Cäsium auf den Träger aufgebracht worden war, enthielt 8,7 Gew.% Silber und 0,017 Gew.% Cäsium.

Unter den Testbedingungen des Beispiels 1 wurden bei 189°C 1,5 Vol.-% Ethylenoxid produziert.

Bei 7% Ethylenumsatz konnte dabei eine Selektivität von 81% erreicht werden.

Beispiel 7

Zur Herstellung eines erfindungsgemässen Katalysators wurden, wie in Beispiel 1 beschrieben, 91,5 g Aluminiumoxidringe in eine Lösung aus

| | |
|---|---|
| 0,900 g (0,090 Gew.%) | Cäsiumnitrat |
| 999,100 g (99,910 Gew.%) | Wasser |

bei Raumtemperatur 5 Minuten lang getaucht. Nach dem Abdekantieren der überschüssigen Lösung wurde der Träger eine Stunde lang bei 110°C getrocknet.

Die auf Raumtemperatur abgekühlten Ringe wurden dann in einer Rührtrommel mit 36 g einer Silberimprägnierlösung aus

| | |
|---|---|
| 10,500 g (26,5823 Gew.%) | Wasser |
| 15,000 g (37,9747 Gew.%; 0,088 mol Silber) | Silbernitrat |
| 3,500 g (8,8607 Gew.%; 0,048 mol) | tert.-Butylamin |
| 10,500 g (26,5823 Gew.%; 0,144 mol) | sec.-Butylamin |

übergossen und 3 Minuten lang bei Raumtemperatur langsam gerührt (es wurden also 0,192 mol erfindungsgemässes Amin aus 25 Gew.% tert.-Butylamin und 75 Gew.% sek.-Butylamin für 0,088 mol Silber, das sind 2,16 mol dieses Amins pro mol Silber, eingesetzt).

Nach 5 Minuten Standzeit wurde der Trommelinhalt 30 Minuten lang bei 110°C getrocknet und anschliessend, wie in Beispiel 1 beschrieben, im Luft-Stickstoff-Strom reduziert.

Der fertige Katalysator, bei dessen Herstellung zuerst das gesamte Cäsium (erste Imprägnierung) und dann (zweite Imprägnierung) das gesamte Silber auf den Träger aufgebracht worden war, enthielt 8,6 Gew.% Silber und 0,018 Gew.% Cäsium.

Dieser Katalysator produzierte unter den Testbedingungen des Beispiels 1 bei 190°C 1,6 Vol.-% Ethylenoxid.

Bei 6% Ethylenumsatz betrug dabei die Selektivität 80,0%.

Beispiel 8

Zur Herstellung eines erfindungsgemässen Katalysators wurde zunächst eine Lösung aus

| | |
|---|---|
| 26,582 g (26,5823 Gew.%) | Wasser |
| 37,975 g (37,9747 Gew.%; 0,223 mol) | Silbernitrat |
| 8,860 g (8,8607 Gew.%; 0,121 mol) | tert.-Butylamin |
| 26,590 g (26,5823 Gew.%; 0,363 mol) | sec.-Butylamin |

bereitet (es wurden also 0,484 mol erfindungsgemässes Amingemisch aus 25 Gew.% tert.-Butylamin und 75 Gew.% sek.-Butylamin für 0,223 mol Silber, das sind 2,17 mol dieses Amins pro mol Silber, eingesetzt).

90 g Trägermaterial gemäss Beispiel 1 wurden in einem ersten Imprägnierschritt in einer drehbaren Kunststofftrommel mit 35,2 g der oben angegebenen Silber-Amin-Imprägnierlösung übergossen. Das Trägermaterial saugte die gesamte Lösung auf. Nun wurde der Trommelinhalt in einem Trockenschrank 40 min lang bei 120°C getrocknet (der Gewichtsverlust betrug 10 g). Mit dieser Behandlung wurden auf dem Träger 25,8 g Silbersalz-Amin-Komplex, das sind 8,5 g Silber, aufgebracht. Nach dem Abkühlen auf Raumtemperatur wurden die Ringe in einer zweiten Imprägnierung in der oben beschriebenen Rührtrommel mit einer Cäsium-Imprägnierlösung aus 38 mg Cäsiumnitrat in 10 g Wasser besprüht, wobei die gesamte Lösung aufgesprüht wurde. Nun wurde bei 120°C getrocknet (der Gewichtsverlust betrug 9 g). Mit diesem zweiten Imprägnierschritt wurde die gesamte Cäsiummenge aufgebracht. Nach dem Abkühlen auf Raumtemperatur wurden in einem dritten Imprägnierschritt 8,6 g der oben angegebenen Silber-Amin-Imprägnierlösung, so wie bei der ersten Imprägnierung aufgebracht, worauf 30 Minuten lang bei 110°C getrocknet wurde.

Der so hergestellte Roh-Katalysator wurde wie in Beispiel 1 beschrieben, mit einem Luft-Stickstoff-Gemisch reduziert. Der fertige Katalysator enthielt 10,6 Gew.% Silber und 0,026 Gew.% Cäsium.

Der Test des Katalysators erfolgte wie in Beispiel 1 und brachte das nachstehende Ergebnis:

| Temperatur °C | Ethylenumsatz Vol.-% | Selektivität % |
|---|---|---|
| 193 | 7 | 81,3 |
| 197 | 10 | 79,5 |

Beispiel 9

Beispiel 8 wurde wiederholt mit dem Unterschied, dass die Reihenfolge der Imprägnierschritte wie folgt geändert wurde:

1. Imprägnierschritt: erster Silberauftrag;
2. Imprägnierschritt: zweiter Silberauftrag;
3. Imprägnierschritt: Cäsiumauftrag.

Der fertige Katalysator enthielt 10,6 Gew.% Silber und 0,026 Gew.% Cäsium.

Der Test des Katalysators erfolgte wie in Beispiel 1 und brachte das nachstehende Ergebnis:

| Temperatur °C | Ethylenumsatz Vol.-% | Selektivität % |
|---|---|---|
| 194 | 7 | 81,1 |
| 198 | 10 | 79,2 |

**Patentansprüche**

1. Silberkatalysator, bestehend aus Silber in einer Menge von 3 bis 20 Gew.% und Kalium, Rubidium, Cäsium oder einer Mischung davon als Promotor in einer Menge von 0,003 bis 0,05 Gew.% auf einem hitzebeständigen porösen Trägermaterial, Gewichtsprozente jeweils bezogen auf das Gewicht des Katalysators, wobei das Silber und der Promotor durch Imprägnieren des Trägermaterials mit Silbersalz-Amin-Komplexen und mit Promotorverbindungen, und durch Trocknen und Erhitzen des imprägnierten Trägermaterials auf 170 bis 400°C zur Zersetzung des Silbersalz-Amin-Komplexes in metallisches Silber aufgebracht worden sind, dadurch gekennzeichnet, dass als Amin zur Bildung des Silbersalz-Amin-Komplexes ein Gemisch aus a) 10 bis 40 Gew.% von mindestens einem tert.-Alkylamin der allgemeinen Formel I

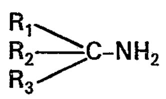

und b) 60 bis 90 Gew.% von mindestens einem sek.-Alkylamin der allgemeinen Formel II

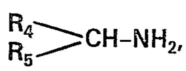

worin $R_1$, $R_2$, $R_3$, $R_4$ und $R_5$ eine Alkylgruppe mit 1 bis 4 Kohlenstoffatomen bedeuten, eingesetzt worden ist.

2. Silberkatalysator nach Anspruch 1, dadurch gekennzeichnet, dass das tert.-Alkylamin tert.-Butylamin und das sek.-Alkylamin sek.-Butylamin ist.

3. Silberkatalysator nach einem oder mehreren der Ansprüche 1 bis 2, dadurch gekennzeichnet, dass die Menge an Silber auf dem Trägermaterial 7 bis 14 Gew.% und die Menge an Promotor 0,008 bis 0,035 Gew.% beträgt.

4. Silberkatalysator nach einem oder mehreren Ansprüchen 1 bis 3, dadurch gekennzeichnet, dass der Promotor Cäsium ist.

5. Verfahren zur Herstellung des Silberkatalysators nach Anspruch 1 bis 4, bei dem auf einem hitzebeständigen, porösen Trägermaterial Silber in einer Menge von 3 bis 20 Gew.% und Kalium, Rubidium, Cäsium oder eine Mischung davon als Promotor in einer Menge von 0,003 bis 0,05 Gew.% aufgebracht werden, Gewichtsprozente jeweils bezogen auf das Gewicht des Katalysators, wobei zum Aufbringen des Silbers und des Promotors das Trägermaterial mit Silbersalz-Amin-Komplexen und mit Promotorverbindungen imprägniert wird und das imprägnierte Trägermaterial zur Zersetzung des Silbersalz-Amin-Komplexes in metallisches Silber auf 170 bis 400°C erhitzt wird, dadurch gekennzeichnet, dass als Amin zur Bildung des Silbersalz-Amin-Komplexes ein Gemisch aus a) 10 bis 40 Gew.% von mindestens einem tert.-Alkylamin der allgemeinen Formel I

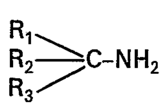

und b) 60 bis 90 Gew.% von mindestens einem sek.-Alkylamin der allgemeinen Formel II

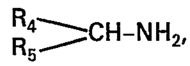

worin $R_1$, $R_2$, $R_3$, $R_4$ und $R_5$ eine Alkylgruppe mit 1 bis 4 Kohlenstoffatomen bedeuten, eingesetzt wird.

6. Verfahren nach Anspruch 5, dadurch gekennzeichnet, dass als tert.-Alkylamin tert.-Butylamin und als sek.-Alkylamin sek.-Butylamin eingesetzt wird.

7. Verfahren nach einem oder mehreren Ansprüchen 5 bis 6, dadurch gekennzeichnet, dass auf das Trägermaterial Silber in einer Menge von 7 bis 14 Gew.% und Promotor in einer Menge von 0,008 bis 0,035 Gew.% aufgebracht werden.

8. Verfahren nach einem oder mehreren Ansprüchen 5 bis 7, dadurch gekennzeichnet, dass als Promotor Cäsium aufgebracht wird.

9. Verwendung des Silberkatalysators nach Anspruch 1 bis 4 zur Herstellung von Ethylenoxid durch direkte Oxidation von Ethylen mit molekularem Sauerstoff.

10. Ausführungsform nach Anspruch 9, dadurch gekennzeichnet, dass sie bei einer Temperatur von 175 bis 250°C unter Anwendung eines Katalysatorfestbettes durchgeführt wird.

**Claims**

1. A silver catalyst which consists of silver in an amount of 3 to 20% by weight and potassium, rubidium cesium or a mixture thereof as promoter in an amount of 0.003 to 0.05% by weight on a heat-resistant porous support material, all the weight percentages being relative to the weight of the catalyst, the silver and the promoter having been applied by impregnating the support material with silver salt/amine complexes and with promoter compounds and by drying and heating the impregnated support material to 170 to 400°C to decompose the silver salt/amine complex into metallic silver, characterised in that the amine used for forming the silver salt/amine complex is a mixture of a) 10 to 40% by weight of at least one tert.-alkylamine of the formula I

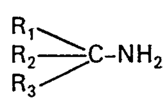

and b) 60 to 90% by weight of at least one sec.-alkylamine of the formula II

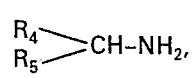

in which $R_1$, $R_2$, $R_3$, $R_4$, and $R_5$ denote an alkyl group having 1 to 4 carbon atoms.

2. The silver catalyst of claim 1, wherein the tert.-alkylamine is tert.-butylamine and the sec.-alkylamine is sec.-butylamine.

3. The silver catalyst as claimed in claims 1 or 2, wherein the amount of silver on the support material is 7 to 14% by weight and the amount of promoter 0.008 to 0.035% by weight.

4. The silver catalyst as claimed in one or more of claims 1 to 3, wherein the promoter is cesium.

5. A process for preparing the silver catalyst of claim 1 by applying to a heat-resistant porous support material silver in an amount of 3 to 20% by weight and potassium, rubidium, cesium or a mixture thereof as promoter in an amount of 0.003 to 0.05% by weight, all the weight percentages being relative to the weight of the catalyst, the silver and the promoter being applied by impregnating the support material with silver salt/amine complexes and with promoter compounds and heating the impregnated support material to 170 to 400°C to decompose the silver salt/amine complex into metallic silver, characterised by using as amine to form the silver salt/amine complex a mixture of a) 10 to 40% by weight of at least one tert.-alkylamine of the formula I

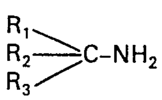

and b) 60 to 90% by weight of at least one sec.-alkylamine of the formula II

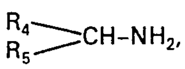

in which $R_1$, $R_2$, $R_3$, $R_4$, and $R_5$ denote an alkyl group having 1 to 4 carbon atoms.

6. The process of claim 5, wherein the tert.-alkylamine used is tert.-butylamine and the sec.-alkylamine is sec.-butylamine.

7. The process as claimed in claims 5 or 6, wherein silver is applied to the support material in an amount of 7 to 14% by weight and the promoter in an amount of 0.008 to 0.035% by weight.

8. The process as claimed in one or more of claims 5 to 7, wherein the promoter applied is cesium.

9. The use of the silver catalyst of claims 1 to 4 for the production of ethylene oxide by direct oxidation of ethylene with molecular oxygen.

10. The use of claim 9, wherein the direct oxidation is carried out at a temperature of 175 to 250°C, the catalyst being arranged as a fixed bed.

**Revendications**

1. Catalyseur à l'argent, constitué d'argent dans une proportion de 3 à 20% en poids et de potassium, de rubidium, de césium ou d'un mélange de ceux-ci comme promoteur dans une proportion de 0,003 à 0,05% en poids sur une matière de support poreuse résistante à la chaleur, les pourcentages pondéraux étant rapportés à chaque fois au poids du catalyseur, l'argent et le promoteur ayant été appliqués par imprégnation de la matière de support avec des complexes sel d'argent-amine et avec des composés promoteurs, et par séchage et chauffage de la matière de support imprégnée à 170 à 400°C pour la décomposition du complexe sel d'argent-amine en argent métallique, caractérisé en ce qu'on a utilisé comme amine pour la formation du complexe sel d'argent-amine un mélange a) de 10 à 40% en poids d'au moins une tert-alkylamine répondant à la formule générale I:

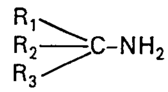

et b) de 60 à 90% en poids d'au moins une sec-alkylamine répondant à la formule générale II:

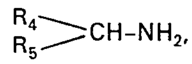

dans lesquelles $R_1$, $R_2$, $R_3$, $R_4$ et $R_5$ désignent un groupe alkyle en $C_1$ à $C_4$.

2. Catalyseur à l'argent suivant la revendication 1, caractérisé en ce que la tert-alkylamine est la tert-butylamine et la sec-alkylamine et la sec-butylamine.

3. Catalyseur à l'argent suivant une ou plusieurs des revendications 1 et 2, caractérisé en ce que la quantité d'argent sur la matière de support est de 7 à 14% en poids et la quantité de promoteur de 0,008 à 0,035% en poids.

4. Catalyseur à l'argent suivant une ou plusieurs des revendications 1 à 3, caractérisé en ce que le promoteur est le césium.

5. Procédé de préparation du catalyseur à l'argent suivant les revendications 1 à 4, dans lequel on applique sur une matière de support résistante à la chaleur poreuse, de l'argent dans une quantité de 3 à 20% en poids et du potassium, du rubidium, du césium ou un mélange de ceux-ci comme promoteur dans une quantité de 0,003 à 0,05% en poids, les pourcentages pondéraux étant rapportés à chaque fois au poids du catalyseur, la matière de support étant imprégnée, pour l'application de l'argent et du promoteur, de complexes sel d'argent-amine et de composés promoteurs, et la matière de support imprégnée étant chauffée, pour la décomposition du complexe sel d'argent-amine en argent métallique, à 170 à 400°C, caractérisé en ce qu'on utilise comme amine pour la formation du complexe sel d'argent-amine un mélange a) de 10 à 40% en poids d'au moins une tert-alkylamine répondant à la formule générale I:

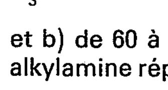

et b) de 60 à 90% en poids d'au moins une sec-alkylamine répondant à la formule générale II:

dans lesquelles $R_1$, $R_2$, $R_3$, $R_4$ et $R_5$ désignent un groupe alkyle en $C_1$ à $C_4$.

6. Procédé suivant la revendication 5, caractérisé en ce qu'on utilise comme tert-alkylamine la tert-butylamine et comme sec-alkylamine la sec-butylamine.

7. Procédé suivant une ou plusieurs des revendications 5 et 6, caractérisé en ce qu'on applique sur la matière de support de l'argent dans une quantité de 7 à 14% en poids, et un promoteur dans une quantité de 0,008 à 0,035% en poids.

8. Procédé suivant une ou plusieurs des revendications 5 à 7, caractérisé en ce qu'on applique comme promoteur du césium.

9. Utilisation du catalyseur à l'argent suivant les revendications 1 à 4 pour la préparation d'oxyde d'éthylène par oxydation directe de l'éthylène avec de l'oxygène moléculaire.

10. Mode de réalisation suivant la revendication 9, caractérisé en ce qu'il est effectué à une température de 175 à 250°C en utilisant un lit fixe de catalyseur.